# EUROPEAN PATENT SPECIFICATION

(11) **EP 4 552 564 B1**
(45) Date of publication and mention of the grant of the patent: **02.12.2026**
(21) Application number: 24207955.6
(22) Date of filing: 22.10.2024
(51) Int. Cl.: A61B 5/11, A61B 5/113, G06T 7/20, G06T 7/00

(54) **MEASUREMENT METHOD, PROGRAM, COMPUTER-READABLE STORAGE MEDIUM, AND MEASUREMENT SYSTEM**
MESSVERFAHREN, PROGRAMM, COMPUTERLESBARES SPEICHERMEDIUM UND MESSSYSTEM
PROCÉDÉ DE MESURE, PROGRAMME, SUPPORT DE STOCKAGE LISIBLE PAR ORDINATEUR ET SYSTÈME DE MESURE

(30) Priority: 10.11.2023 JP 2023192188
(43) Date of publication of application: 14.05.2025
(73) Proprietor: SHIMADZU CORPORATION, Kyoto-shi, Kyoto 604-8511 (JP)
(72) Inventor: KARASAWA, Tomohiro, Kyoto-shi, Kyoto, 604-8511 (JP); YOSHIDA, Koki, Kyoto-shi, Kyoto, 604-8511 (JP); KAMON, Masamitsu, Kyoto-shi, Kyoto, 604-8511 (JP); FURUTA, Masafumi, Kyoto-shi, Kyoto, 604-8511 (JP)
(74) Representative: Müller-Boré & Partner Patentanwälte PartG mbB

(56) References cited:
- EP-A1- 2 772 189
- US-A1- 2017 238 805
- US-A1- 2020 046 302

## Description

### BACKGROUND OF THE INVENTION

### Field of the Invention

The present invention relates to a measurement method, a program, and a measurement system, and more particularly to a technique to improve accuracy in measurement of biological information.

### Description of the Background Art

Biological information is used as an index of a current health condition of a subject, used for diagnosis of a disease, and used for determination of a therapeutic effect. In order to find change in physical condition early and to treat any disease early, needs for measurement of biological information in a living condition at home have increased.

In daily measurement of biological information, a contactless measurement method without a side effect such as uncomfortableness and skin irritation caused by attachment of a measurement instrument is preferred for a subject. In this connection, Japanese National Patent Publication No. 2016-522027 discloses a technique to detect a vital sign from a frame image group obtained by imaging of a subject.

In obtaining biological information based on moving images obtained by imaging of a subject, a motion derived from the biological information may be small and sensitivity in measurement of the biological information of the subject may be insufficient. In this connection, Shoichiro Takeda, Megumi Isogai, Shinya Shimizu, and Hideaki Kimata, "Local Riesz Pyramid for Faster Phase-Based Video Magnification," June 2020, IEICE Transactions on Information and Systems discloses a technique to emphasize small change in video images.

Document US 2017/238805 A1 describes a video-based method for monitoring vital signs, wherein images of a patient are captured, intensity signals are produced from the images, signals are filtered to focus on a physiologic component, and a vital sign is measured from the filtered signals.

Document US 2020/046302 A1 describes a system and a method for calculating a change in depth of regions in one or more regions of interest (ROI's) on a patient and assigning one or more visual indicators to the regions based on the calculated changes in depth of the regions over time. Particularly, one or more breathing parameter signals corresponding to the regions can be generated and/or analyzed. The one or more visual indicators can be displayed overlaid onto the regions in real-time. The system and method can also display one or more generated breathing parameter signals in real-time and/or can trigger an alert and/or an alarm when a breathing abnormality is detected.

Document EP 2 772 189 A1 describes an apparatus for determining vital sign information from a subject, comprising a detection unit for detecting radiation from a field of view and for determining characteristic parameter including vital sign information of the subject from different areas of the field of view, a frequency analysis unit for determining a spectral parameter of the characteristic parameter derived from the different areas, a selection unit for selecting at least one of the areas of the field of view on the basis of the spectral parameter, and a calculation unit for calculating the vital sign information on the basis of the characteristic parameter from the at least one selected area.

### SUMMARY OF THE INVENTION

Though the biological information can be obtained from the frame image group obtained by imaging of the subject with the technique disclosed in Japanese National Patent Publication No. 2016-522027, the motion derived from the biological information may be minor and the biological information may not be measured at sufficient measurement sensitivity.

Though a small motion in video images can be emphasized with the technique disclosed in Shoichiro Takeda, Megumi Isogai, Shinya Shimizu, and Hideaki Kimata, "Local Riesz Pyramid for Faster Phase-Based Video Magnification," June 2020, IEICE Transactions on Information and Systems, not only the motion derived from biological information of interest but also a motion irrelevant to the biological information of interest may be emphasized. In such a case, noise becomes great and the biological information may not be obtained at sufficient measurement accuracy.

The present invention was invented in view of such circumstances, and an object thereof is to provide a technique to improve accuracy in measurement of biological information. The present invention is defined by the subject-matter of the independent claims. Preferred embodiments are defined in the dependent claims.

A measurement method according to a first aspect of the present disclosure is a measurement method of measuring biological information from a frame image group obtained by imaging over time of a subject, and includes dividing a target region in each frame image included in the frame image group into a plurality of sub regions, extracting an amount of variation in each of the plurality of sub regions from the frame image group, determining sub regions similar in amount of variation from among the plurality of sub regions, and obtaining the biological information of the subject based on a summed value of the amount of variation in each of the similar sub regions.

A measurement system according to a second aspect of the present disclosure includes a camera that images a subject over time and generates a frame image group and a processing apparatus that performs processing for measuring biological information of the subject from the frame image group generated by the camera. The processing apparatus is configured to divide a target region in each frame image included in the frame image group into a plurality of sub regions, extract an amount of variation in each of the plurality of sub regions from the frame image group, determine sub regions similar in amount of variation from among the plurality of sub regions, and obtain the biological information of the subject based on a summed value of the amount of variation in each of the similar sub regions.

The foregoing and other objects, features, aspects and advantages of this invention will become more apparent from the following detailed description of this invention when taken in conjunction with the accompanying drawings.

### BRIEF DESCRIPTION OF THE DRAWINGS

Fig. 1 is a schematic configuration diagram of a measurement system.
Fig. 2 is a diagram showing a hardware configuration of a processing system.
Fig. 3 is a diagram for illustrating a method of identifying sub regions similar in amount of variation and obtaining an amount of variation in a target region.
Fig. 4 is a flowchart showing processing involved with measurement of biological information.
Fig. 5 is a diagram for illustrating a method of setting a target region.
Fig. 6 is a diagram for illustrating a method of outputting a position of a landmark, with a frame image being defined as input.
Fig. 7 is a diagram for illustrating a method of setting a target region in a doughnut shape within a certain angular range, with the position of the landmark being defined as a rotation center.

### DESCRIPTION OF THE PREFERRED EMBODIMENTS

An embodiment of the present invention will be described in detail with reference to the drawings. The same or corresponding elements in the drawings have the same reference characters allotted and description thereof will not be repeated.

### [Overall Configuration of Measurement System]

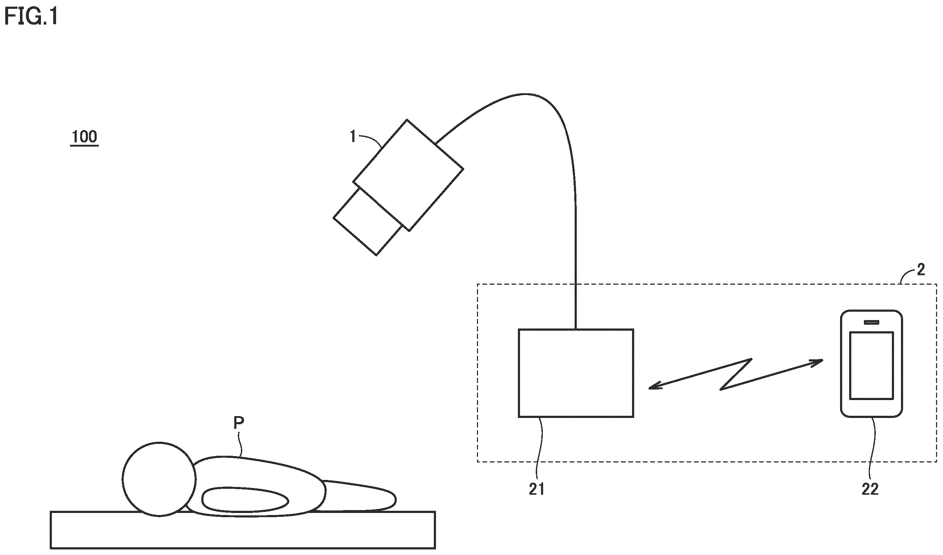

Fig. 1 is a diagram schematically showing an overall configuration of a measurement system 100 according to the present embodiment. As shown in Fig. 1, measurement system 100 includes a camera 1 and a processing system 2. Measurement system 100 obtains biological information of a subject P, for example, from data obtained by imaging of subject P who is sleeping.

Camera 1 is arranged to accommodate subject P within a field of view of imaging. Camera 1 includes an optical system such as a lens and an image pick-up element. The image pick-up element is implemented, for example, by a charge coupled device (CCD) sensor and a complementary metal oxide semiconductor (CMOS) sensor. The image pick-up element generates imaging data by converting light incident from subject P through the optical system into an electrical signal. Camera 1 may be stationary, may be adjustable in angle of image pick-up or zoom by remote control, or may be a depth camera capable of obtaining three-dimensional information.

Processing system 2 includes a main body apparatus 21 and a terminal 22. Processing system 2 analyzes imaging data obtained by camera 1 to obtain biological information of subject P, and shows the obtained biological information to a measurer.

In this embodiment, main body apparatus 21 is communicatively connected to camera 1 through a wire or wirelessly. Main body apparatus 21 is implemented, for example, by a general-purpose computer such as a desktop computer. Terminal 22 is connected to main body apparatus 21 to communicate with each other, for example, over the Internet. Terminal 22 is implemented, for example, by a smartphone and a tablet terminal. A hardware configuration of main body apparatus 21 and terminal 22 will be described later.

Main body apparatus 21 and terminal 22 may physically be integrated, for example, like a personal computer or a tablet, or a server system interposed between main body apparatus 21 and terminal 22 may further be provided. Processing system 2 may be integrated with camera 1. Camera 1 and processing system 2 are not limited in terms of physical aspect thereof, so long as an equivalent function is exhibited.

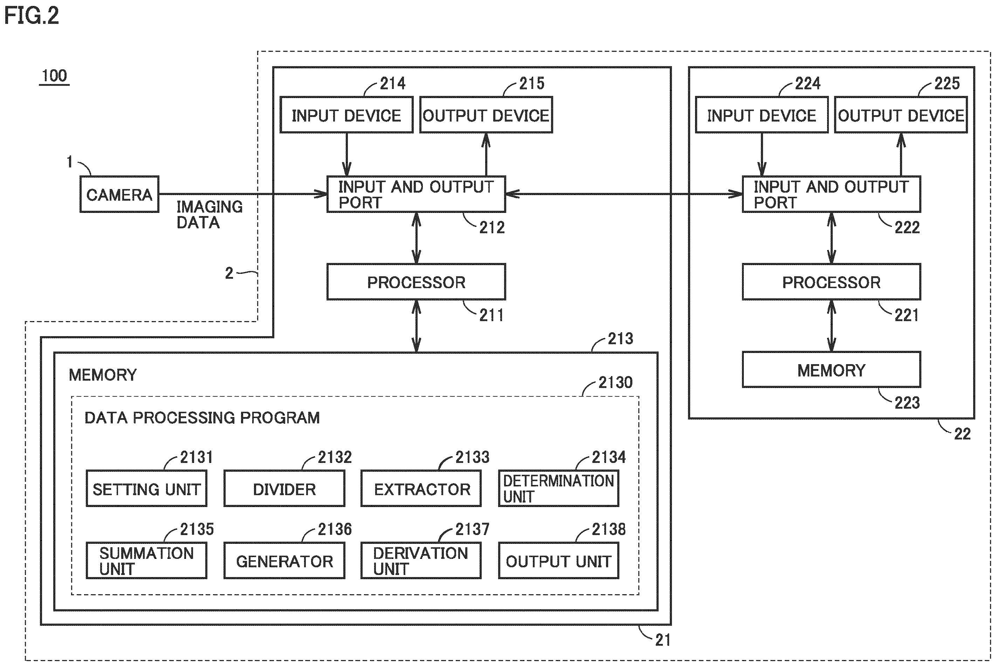

Fig. 2 is a diagram showing a hardware configuration of processing system 2. The hardware configuration of each of main body apparatus 21 and terminal 22 will be described with reference to Fig. 2.

Main body apparatus 21 includes a processor 211, an input and output port 212, a memory 213, an input device 214, and an output device 215.

Processor 211 is exemplary electric circuitry and controls an operation of main body apparatus 21 by executing a given program. The program executed by processor 211 may be stored in memory 213 or in a storage outside main body apparatus 21.
The processor is implemented, for example, by a central processing unit (CPU).

Data is stored in memory 213. Data stored in memory 213 includes a data processing program 2130. Memory 213 includes a volatile memory (for example, a random access memory (RAM)) and a non-volatile memory (for example, a read only memory (ROM), a hard disk drive, and a solid state drive).

Data processing program 2130 is a program for obtaining biological information from imaging data. Data processing program 2130 may be stored in memory 213 or in an external storage accessible from processor 211.

By being executed by processor 211, data processing program 2130 allows processor 211 to perform at least eight functions (a setting unit 2131, a divider 2132, an extractor 2133, a determination unit 2134, a summation unit 2135, a generator 2136, a derivation unit 2137, and an output unit 2138). In one implementation, data processing program 2130 includes a module for each function. Contents of each function will be described later.

Input device 214 accepts input of information to main body apparatus 21. The information is, for example, a region where a target region is set. Input device 214 is implemented, for example, by a mouse and a keyboard.

Output device 215 outputs information in accordance with an instruction from processor 211. The information is, for example, imaging data obtained by camera 1 and biological information of subject P. Output device 215 is implemented, for example, by a display and a speaker.

Input of information to main body apparatus 21 and output of information from main body apparatus 21 may be provided by terminal 22.

Terminal 22 includes a processor 221, an input and output port 222, a memory 223, an input device 224, and an output device 225.

Processor 221 is exemplary electric circuitry and controls an operation of terminal 22 by executing a given program. The program executed by processor 221 may be stored in memory 223 or in a storage outside terminal 22. The processor is implemented, for example, by a CPU.

Data is stored in memory 223. Data stored in memory 223 is, for example, data obtained by imaging by camera 1 and biological information obtained by main body apparatus 21. Memory 223 includes a volatile memory (for example, a RAM) and a non-volatile memory (for example, a ROM, a hard disk drive, and a solid state drive).

Input device 224 accepts input of information to terminal 22. The information is, for example, a region where a target region is set. Input device 224 is implemented, for example, by a mouse, a keyboard, and a touch panel.

Output device 225 outputs information in accordance with an instruction from processor 221. The information is, for example, imaging data obtained by camera 1 and biological information of subject P. Output device 225 is implemented, for example, by a display and a speaker.

### [Comparative Example]

A method of contactless measurement of biological information is preferred for a subject, because there is no side effect such as uncomfortableness and skin irritation caused by attachment of a measurement instrument. A method of obtaining biological information based on data obtained by imaging with a camera is available as the method of contactless measurement of biological information. For example, a state of a sleeping subject is imaged and respiratory information of the subject is obtained from the obtained imaging data.

In such a method, an object other than the subject may be included in imaging data. Then, a method of setting a region corresponding to the subject or a part of the body of the subject as a target region in the imaging data and obtaining biological information based on a motion of the subject in the target region may be used.

Even in such a case, the motion of the body on which the biological information is based may be minor and sensitivity in measurement of the biological information may be insufficient. There is also a method of augmenting a signal based on the biological information by performing processing for emphasizing minor change in imaging data with a technique such as what is called video magnification. With this method, however, a signal (noise) other than the signal based on the biological information may be augmented, and accuracy in measurement of the biological information may be insufficient.

### [Measurement of Biological Information According to Embodiment]

Then, in measurement of the biological information according to the present embodiment, a target region in each frame image included in a frame image group obtained by imaging over time is divided into a plurality of sub regions, and only sub regions similar in amount of variation are selected from among the plurality of sub regions. The amount of variation is then calculated based on data in the selected sub regions. With this method, only signals in the sub regions selected as above from the target region are used for measurement of the biological information, and hence accuracy in measurement of the biological information can be improved. Since the signal based on the biological information and noise are distinguished from each other also in imaging data subjected to the processing for emphasizing minor change and the amount of variation in the target region is calculated only based on the signal based on the biological information, an S/N ratio in measurement of the biological information can be improved.

### [Imaging Data Used for Measurement of Biological Information]

Imaging data obtained by camera 1 and used for measurement of biological information of subject P will be described.

Imaging data is a frame image group composed of a plurality of successively obtained frame images. Though a time interval in obtaining frame images is not particularly limited, it is preferably shorter than a cycle of biological information of interest. Specifically, the interval in obtaining frame images is set, for example, to thirty-three milliseconds.

In one implementation, the subject is accommodated in a frame image of imaging data. A posture of the subject is not particularly limited, and the posture may be a lying posture, a standing posture, or a seated posture. In the case of the lying posture, lying on the back, lying laterally, or lying on the stomach may be applicable. The imaging data may be obtained by imaging of the subject who is sleeping, the subject at rest, the subject who is active, or the subject who undergoes a medical test (for example, nuclear magnetic resonance imaging and computed tomography scan). Being at rest refers to a state in which the subject is awake and not sleeping but there is no continuous body motion of the subject. Therefore, when the subject is at rest, positions of the subject and the camera relative to each other do not substantially change.

The biological information is periodic and represented as waveform information. The biological information is, for example, respiratory information. The respiratory information defines at least one of a rate of respiration, a respiration time period, an expiration time period, an inspiration time period, an amplitude, a time period from expiration to start of inspiration, and a tidal volume. The respiration time period is a total of the expiration time period and the inspiration time period.

Raw data or data subjected to given processing (for example, processing by a technique called video magnification (for example, Riesz pyramid)) may be used as the imaging data.

At the time point of start of processing for obtaining biological information which will be described later, all frame images in imaging data to be analyzed may have been or may not have been obtained.

### [Method of Measuring Biological Information]

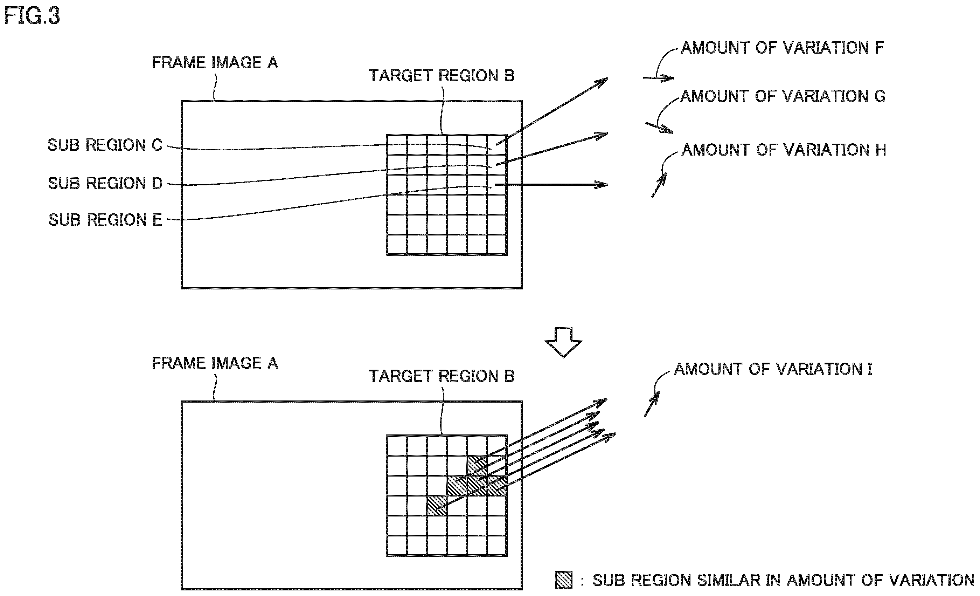

A method of measuring biological information based on obtained imaging data will be described. Fig. 3 is a diagram for illustrating a method of obtaining biological information from imaging data obtained by camera 1. In Fig. 3, a frame image A represents one frame image obtained at certain timing included in the imaging data. In Fig. 3, a target region B represents a region to be used for measurement of biological information in frame image A.

### <1. Setting of Target Region>

Setting of the target region by setting unit 2131 will be described. The target region is a region in the frame image to be used for measurement of biological information. The target region may be determined in advance or designated by the measurer. The target region determined in advance means, for example, that the entire frame image is to be processed as the target region and a portion provided with a characteristic design of apparel that subject P wears is defined as the target region. The target region may be designated by the measurer through input device 214 of main body apparatus 21 or input device 224 of terminal 22. An amount of calculation required for measurement of the biological information can be reduced by setting a part of the frame image as the target region.

### <2. Division of Target Region>

Division of the target region by divider 2132 will be described. Divider 2132 divides the target region into a plurality of sub regions. The sub region may be defined by a unit of one pixel or a unit of a plurality of pixels. When the sub region is defined by the unit of one pixel, accuracy in measurement of biological information is improved, whereas a processing time period for measurement of the biological information may be longer due to increase in amount of calculation. When the sub region is defined by the unit of a plurality of pixels, on the other hand, a shorter processing time period for measurement of the biological information is expected owing to reduction in amount of calculation, whereas accuracy in measurement of the biological information may be lower. Therefore, the measurer selects as appropriate, the unit of division of the target region based on relation between accuracy in measurement of the biological information desired by a user and the processing time period required for measurement. A position and a range of the sub region in each frame image are common among a plurality of frame images when the target region is the same.

In the example in Fig. 3, target region B is divided into 6×6 or thirty-six sub regions including sub regions C, D, and E. The number of sub regions is not limited as shown in Fig. 3, and may be set as appropriate for each situation where the measurement system is implemented.

### <3. Extraction of Amount of Variation>

Extraction of an amount of variation in the sub region by extractor 2133 will be described. Extractor 2133 extracts the amount of variation in each of the plurality of sub regions generated by division of the target region.

The amount of variation is, for example, the amount of variation of a gradient value in the sub region. Physical variation in position of a part of the body of the subject may vary the gradient value in the sub region corresponding to the part. The amount of variation extracted for the sub region in the imaging data thus means a physical amount of variation.

The amount of variation is calculated, for example, as a difference between the gradient value in the sub region in the frame image defined as the reference and the gradient value in the sub region in the frame image of interest. Alternatively, the amount of variation may be calculated as a difference between an average value or a median value of the gradient values in the sub regions in a plurality of prescribed frame images and the gradient value in the sub region in the frame image of interest.

The amount of variation may be an amount of variation of a phase based on a phase signal of each sub region taken out by frequency analysis.

In the example in Fig. 3, extractor 2133 extracts the amount of variation in each sub region. Fig. 3 shows the amounts of variation extracted from sub regions C, D, and E as respective amounts of variation F, G, and H.

### <4. Determination of Sub Region Group>

Determination of a sub region group by determination unit 2134 will be described. Determination unit 2134 selects sub regions similar in amount of variation to each other as the sub region group. In one implementation, the amounts of variation in two sub regions being similar to each other means that a difference in amount of variation between the two sub regions is equal to or smaller than a prescribed value. The prescribed value may be fixed or changed for each piece of imaging data. Determination as to whether or not the sub regions are similar may be made based on whether or not a Euclidean distance between phase signals of sub regions is equal to or smaller than a given value.

In the example in Fig. 3, the sub region group is determined from among thirty-six sub regions. Fig. 3 shows five sub regions included in the sub region group with hatching.

In an example where a frame image includes a stationary object in a region other than the subject, in sub regions corresponding to the object, gradient values are not substantially different between frame images. Though a plurality of sub regions corresponding to such an object are similar to each other in amount of variation, selection of those sub regions as the sub regions to be included in the sub region group is preferably avoided. For example, sub regions having an amount of variation between frame images equal to or smaller than a prescribed value may be excluded from processing for determining the sub region group. The prescribed value may be fixed or changed for each piece of imaging data.

For a similar purpose, determination unit 2134 may normalize the phase signal obtained from each sub region, and when variance of the normalized phase signal is smaller than a prescribed value, determination unit 2134 may exclude the sub region from which the phase signal is derived, from processing for determining the sub region group. The prescribed value may be fixed or changed for each piece of imaging data.

Determination unit 2134 may have a process return to setting of the target region when the number of sub regions included in the sub region group is smaller than a prescribed number. This is because, when the number of sub regions included in the sub region group is smaller than the prescribed number, for example, subject P may have moved out of a range of the set target region. Therefore, the target region should newly be set at a position different from the set target region. In other words, when the number of sub regions included in the sub region group is smaller than the prescribed number, setting unit 2131 newly sets the target region. The prescribed value may be fixed or changed for each piece of imaging data.

### <5. Summation of Amounts of Variation>

Summation of amounts of variation by summation unit 2135 will be described. Summation unit 2135 sums the amount of variation in each of the sub region included in the sub region group to calculate the amount of variation of the frame image. In one implementation, an average or a median value of the amounts of variation in the plurality of sub regions included in the sub region group is calculated as the amount of variation in the frame image.

Fig. 3 shows an amount of variation I calculated based only on the amounts of variation in five sub regions included in the sub region group, as the amount of variation in frame image A.

### <6. Generation of Waveform Data>

Generation of waveform data by generator 2136 will be described. As processing in 1. to 5. described above is performed on a plurality of frame images, the amount of variation in each frame image is calculated. Generator 2136 generates waveform data based on the amount of variation in each frame image in the imaging data.

### <7. Derivation of Biological Information>

Derivation of biological information by derivation unit 2137 will be described. Derivation unit 2137 derives a result of measurement of the biological information based on generated waveform data. For example, the result of measurement of biological information is derived based on a cycle of the waveform data.

### <8. Output of Biological Information>

Output of biological information by output unit 2138 will be described. The measured biological information is shown on output device 215 and/or output device 225. The waveform data may be shown together with the measured biological information.

### [Process Flow]

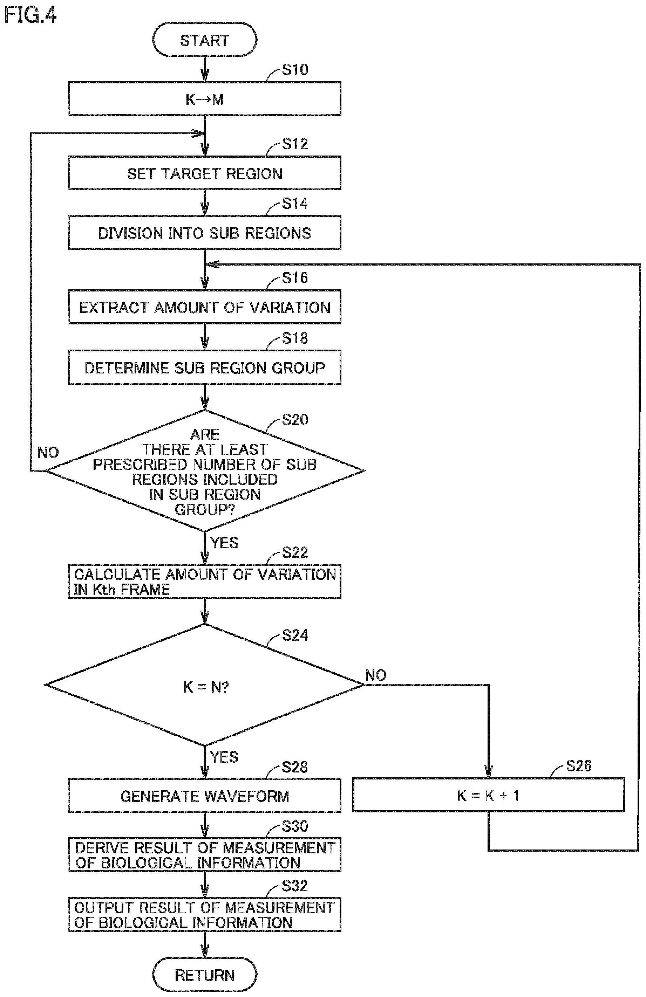

Fig. 4 is a flowchart of processing performed for measurement of biological information in processing system 2. In one implementation, processing in Fig. 4 is performed as being called from a main routine when processor 211 executes a given program. The imaging data is a frame image group composed of L frame images obtained by imaging over time. Processor 211 measures the biological information based on Mth to Nth frame images (M and N being integers satisfying relation of 1 ≤ M < N ≤ L) of L frame images. Values of M and N may be determined in advance or inputted by the user in execution of the given program by processor 211. The imaging data may not have completely been obtained at the time point of start of the processing in Fig. 4. In other words, processor 211 may perform the processing in Fig. 4 on frame images successively obtained by camera 1.

In step S10, processing system 2 sets a value of a variable K to be used in the processing in Fig. 4 to M.

In step S12, processing system 2 sets the target region in a Kth frame image in imaging data received from camera 1, for example, as described above as the function of setting unit 2131.

In step S14, processing system 2 divides the target region set in step S12 into a plurality of sub regions, for example, as described above as the function of divider 2132.

In step S16, processing system 2 extracts the amount of variation in each of the plurality of sub regions generated in step S14, for example, as described above as the function of extractor 2133.

In step S18, processing system 2 determines the sub region group, that is, selects sub regions similar to each other in amount of variation, for example, as described above as the function of determination unit 2134.

In step S20, processing system 2 determines whether or not the number of sub regions included in the sub region group determined in step S18 is equal to or larger than a prescribed number. The prescribed number is set to at least two. When the number of identified sub regions is equal to or larger than the prescribed number (YES in step S20), processing system 2 has control proceed to step S22, and otherwise (NO in step S20), processing system 2 has control return to step S12.

Determination in step S20 may be made based on a ratio of the number of selected sub regions to the total number of sub regions. In this case, when the ratio is equal to or larger than a prescribed value, processing system 2 has control proceed to step S22, and otherwise, processing system 2 has control return to step S12.

In step S22, processing system 2 sums the amounts of variation in the similar sub regions and calculates the amount of variation in the Kth frame image, for example, as described above as the function of summation unit 2135.

In step S24, processing system 2 determines whether or not the value of variable K has reached N above. When the value of variable K has reached N above (YES in step S24), processing system 2 has control proceed to step S28, and otherwise (NO in step S24), processing system 2 has control proceed to step S26.

In step S26, processing system 2 increments the value of variable K by one and has control return to step S16.

In step S28, processing system 2 calculates the waveform data based on the amounts of variation in the Mth frame image to the Nth frame image, for example, as described above as the function of generator 2136.

In step S30, processing system 2 derives the result of measurement of the biological information desired by the measurer based on the waveform data obtained in step S28, for example, as described above as the function of derivation unit 2137.

In step S32, processing system 2 outputs the biological information derived in step S30 to output device 215 and/or output device 225, for example, as described above as the function of output unit 2138. Thereafter, processing system 2 quits a biological information measurement subroutine and has the process return to the main routine.

In the method of measuring biological information described above, only a region including variation derived from the biological information among the sub regions resulting from division of the target region is selectively used for measurement of the biological information. Accuracy in measurement of the biological information can thus be improved.

In addition, as the target region is set, the amount of calculation can be reduced. Furthermore, by exclusion of a region not containing a signal originating from the biological information from the target region, measurement of noise can be prevented and the S/N ratio in measurement of the biological information can be improved.

When a frame image is newly obtained after the biological information is obtained, the biological information may be updated based on the amount of variation included in the newly obtained frame image. For example, an example where the biological information is measured based on the imaging data composed of L frame images obtained by imaging over time is assumed. When processing system 2 obtains an L+1th frame image after the frame image obtained in the Lth place, processing system 2 performs processing from step S12 to step S22 on the L+1th frame image and calculates the amount of variation in the L+1th frame image. Processing system 2 generates new waveform data based on the calculated amount of variation in the L+1th frame image and the previously generated waveform data. Processing system 2 updates the biological information based on the newly generated waveform data. The updated biological information is based on the imaging data composed of L+1 frame images obtained by imaging over time.

For example, when the result of measurement of the biological information is derived in parallel to obtainment of the imaging data, the biological information is updated based on the frame image added to the imaging data, so that the latest biological information of the subject can be provided to the measurer.

### [Other Embodiments Involved with Setting of Target Region]

Specific examples of other embodiments involved with setting of the target region will be described.

### <Example 1>

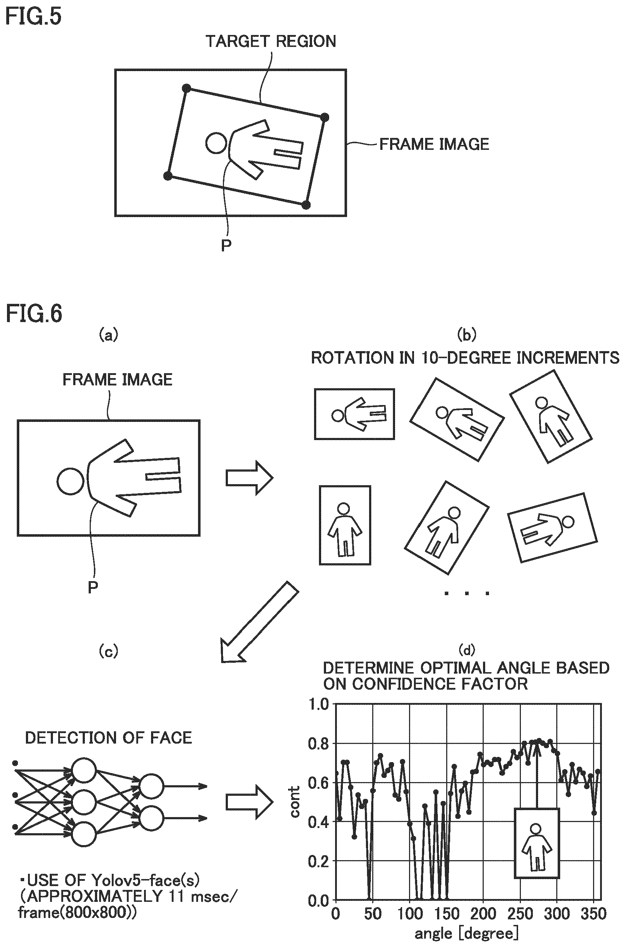

Fig. 5 is a diagram for illustrating a method of setting the target region. In Fig. 5, the frame image includes the whole body of subject P. In such a case, in the frame image shown on output device 215 and/or output device 225, the target region is set as a rectangular region set by the measurer to include the entirety of subject P. This setting is made through input device 214 and/or input device 224.

Instead of input by the measurer, processing system 2 may automatically set the target region. For example, processing system 2 identifies a region where subject P is shown, based on image recognition by machine learning such as semantic segmentation, and automatically sets an appropriate region of a certain shape such as a rectangular shape including this region as the target region. A size or a position of the target region in the entire frame image may be varied for each frame image depending on a motion or the like of the subject. The size of the target region is preferably set to be equal in ratio to the size of shown subject P. This target region is not limited to the rectangular region or the like, and it may be in a shape in a unit of pixels.

### <Example 2>

A method of setting a target region based on a landmark in a frame image will be described. Processing system 2 detects a landmark in the frame image and sets the target region with the landmark being defined as the reference.

For example, a face or a neck of the subject may be adopted as the landmark. A position of the landmark may be determined based on input by the measurer or based on a trained model (for example, Yolo-v5face).

Fig. 6 (a) to (d) shows specific examples. While processing system 2 rotates (shown in Fig. 6 (b)) a frame image (shown in Fig. 6 (a)) at a certain angle in the plane, it estimates a position of the landmark at a plurality of angles based on machine learning (shown in Fig. 6 (c)) and adopts a result of detection of the position of the landmark (shown in Fig. 6 (d)) at an angle highest in confidence factor.

In an example where the face is adopted as the landmark and respiratory information is defined as the biological information, processing system 2 determines a region of a chest portion or an abdominal portion based on a position of a face region including the face defined as the landmark. Processing system 2 may extract the respiratory information as the biological information based on an estimated motion of the region of the chest portion or the abdominal portion. When the face is inclined with respect to a body axis, an actual position of the region of the chest portion or the abdominal portion may be displaced from the determined position. Processing system 2 may then determine a region around the determined position of the region of the chest portion or the abdominal portion as the target region, and may extract the respiratory information as the biological information for this target region.

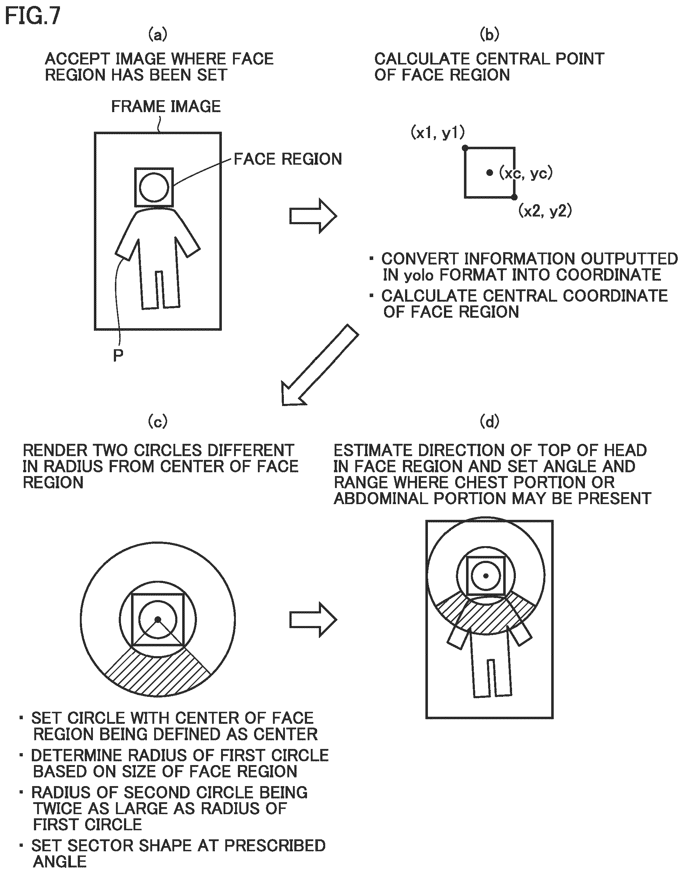

The target region may be set in a doughnut shape within a certain angular range, with the determined position of the landmark being defined as a rotation center. A specific example will be described with reference to Fig. 7. Processing system 2 calculates a face region from a result of detection of the face in an angular image high in confidence factor (Fig. 7 (a)) and calculates a central point thereof (Fig. 7 (b)). Processing system 2 then draws two concentric circles around the central point and sets a sector shape at a certain angle in a doughnut region surrounded by the double concentric circles (Fig. 7 (c)). Processing system 2 applies this shape to the angular image high in confidence factor, and sets a range of the sector shape as a range where the chest portion or the abdominal portion may be present (Fig. 7 (d)).

A position and a size of the chest portion and the abdominal portion of subject P may be estimated based on a face feature value which is a feature value obtained from an image of the face of subject P. The face feature value is, for example, a distance between two face feature points as well as an average of brightness, a brightness histogram, and an area of a triangular region drawn by connection of three face feature points. The face feature points refer to a plurality of points set on the face. The face feature point is set, for example, at the inner corner of the eye, the outer corner of the eye, the pupil, the head of the eyebrow, the peak of the eyebrow, the tail of the eyebrow, the nasal apex, the nasal wing, the nasal root, and/or the corner of the mouth. Alternatively, the position and the size of the chest portion and the abdominal portion of subject P may be estimated based on a skeletal feature value obtained from an image of the whole body. The skeletal feature value refers, for example, to an average of brightness, a brightness histogram, and an area of a region surrounded by feature points in the shoulder, the lower back, and the neck estimated from the image of the whole body.

As the target region is thus limited, the amount of calculation involved with measurement of the biological information can be reduced.

It should be understood that the embodiment disclosed herein is illustrative and non-restrictive in every respect. The scope of the present disclosure is defined by the terms of the claims rather than the description of the embodiment above and is intended to include any modifications within the scope of the claims. Each technique in the embodiment is intended to be carried out alone or in combination with another technique in the embodiment as much as possible as necessary.

Though an embodiment of the present invention has been described, it should be understood that the embodiment disclosed herein is illustrative and non-restrictive in every respect. The scope of the present invention is defined by the terms of the claims and is intended to include any modifications within the scope of the claims.

## Claims

1. A computer-implemented measurement method of measuring periodic biological information from a frame image group obtained by imaging over time of a subject (P), the measurement method comprising:
dividing a target region (B) in each frame image (A) included in the frame image group into a plurality of sub regions (C, D, E);
extracting an amount of variation (F, G, H) in each of the plurality of sub regions (C, D, E) from the frame image group;
determining sub regions similar in amount of variation (F, G, H) to each other from among the plurality of sub regions (C, D, E), wherein sub regions having an amount of variation between frame images equal to or smaller than a prescribed value being excluded from processing for determining a sub region group; and
obtaining the periodic biological information of the subject (P) based on a summed value of the amount of variation (I) in each of the similar sub regions.

2. The measurement method according to claim 1, wherein
the frame image (A) includes the subject (P) who is sleeping.

3. The measurement method according to claim 1, wherein
the frame image (A) includes the subject at rest.

4. The measurement method according to any one of claims 1 to 3, wherein
the biological information is respiratory information.

5. The measurement method according to claim 4, wherein
the respiratory information includes at least one of a rate of respiration, a respiration time period, an expiration time period, an inspiration time period, an amplitude, a time period from expiration to start of inspiration, and a tidal volume.

6. The measurement method according to any one of claims 1 to 5, wherein
the sub region (C, D, E) is composed of one pixel or a plurality of pixels.

7. The measurement method according to any one of claims 1 to 6, further comprising setting the target region (B) in the frame image (A).

8. The measurement method according to claim 7, further comprising:
determining whether the target region (B) includes at least a prescribed number of sub regions similar in amount of variation; and
setting the target region (B) in response to determination that the target region (B) does not include at least the prescribed number of sub regions similar in amount of variation.

9. The measurement method according to claim 7 or claim 8, further comprising estimating a position and a size of a chest portion or an abdominal portion of the subject (P) based on a position and an orientation of a head of the subject (P), wherein
the setting the target region (B) includes setting as the target region (B), a region including the chest portion or the abdominal portion based on the position and the size of the chest portion or the abdominal portion.

10. The measurement method according to claim 9, wherein
the estimating a position and a size includes setting the position and the size of the chest portion or the abdominal portion of the subject based on a feature value of a face of the subject (P).

11. A program executed by a processor (211) mounted on a computer (21), the program causing the computer (21) to perform the measurement method according to any one of claims 1 to 10.

12. A computer-readable storage medium storing a program which, when executed by a computer (21), makes the computer execute the measurement method according to any one of claims 1 to 10.

13. A measurement system (100) comprising:
a camera (1) configured to image a subject (P) over time and to generate a frame image group; and
a processing apparatus (2) configured to perform processing for measuring periodic biological information of the subject (P) from the frame image group generated by the camera (1), wherein
the processing apparatus (2) is configured to
divide a target region (B) in each frame image (A) included in the frame image group into a plurality of sub regions (C, D, E),
extract an amount of variation (F, G, H) in each of the plurality of sub regions (C, D, E) from the frame image group,
determine sub regions (C, D, E) similar in amount of variation (F, G, H) to each other from among the plurality of sub regions (C, D, E), wherein sub regions having an amount of variation between frame images equal to or smaller than a prescribed value being excluded from processing for determining a sub region group, and
obtain the periodic biological information of the subject (P) based on a summed value (I) of the amount of variation in each of the similar sub regions.

## Patentansprüche

1. Ein computerimplementiertes Messverfahren zum Messen periodischer biologischer Informationen aus einer Einzelbildgruppe, die durch zeitlich erfolgende Bildaufnahme eines Subjekts (P) gewonnen wurden, wobei das Verfahren Folgendes umfasst:
Unterteilen eines Zielbereichs (B) in jedem der in der Einzelbildgruppe enthaltenen Einzelbilder (A) in eine Vielzahl von Teilbereichen (C, D, E);
Extrahieren eines Veränderungsbetrags (F, G, H) in jedem der Vielzahl von Teilbereichen (C, D, E) aus der Einzelbildgruppe;
Bestimmen von Teilbereichen, aus der Vielzahl von Teilbereichen (C, D, E), die hinsichtlich des Veränderungsbetrags (F, G, H) einander ähnlich sind, wobei Teilbereiche, deren Veränderungsbetrag zwischen den Einzelbildern gleich oder kleiner als ein vorgegebener Wert ist, von der Verarbeitung zur Bestimmung einer Teilbereichsgruppe ausgeschlossen werden; und
Erhalten der periodischen biologischen Information des Subjekts (P) auf der Grundlage eines Summenwerts des Veränderungsbetrags (I) in jedem der ähnlichen Teilbereiche.

2. Das Verfahren nach Anspruch 1, wobei
das Einzelbild (A) das schlafende Subjekt (P) beinhaltet.

3. Das Verfahren nach Anspruch 1, wobei
das Einzelbild (A) das Subjekt in Ruhe beinhaltet.

4. Das Messverfahren nach irgendeinem der Ansprüche von 1 bis 3, wobei
die biologischen Informationen Ateminformationen sind.

5. Das Messverfahren nach Anspruch 4, wobei
die Ateminformationen mindestens eines von einer Atemfrequenz, einer Atemdauer, einer Ausatmungsdauer, einer Einatmungsdauer, einer Amplitude, einer Zeitspanne von der Ausatmung bis zum Beginn der Einatmung und einem Atemvolumen umfassen.

6. Das Messverfahren nach irgendeinem der Ansprüche von 1 bis 5, wobei
der Teilbereich (C, D, E) aus einem Pixel oder einer Vielzahl von Pixeln besteht.

7. Das Messverfahren nach irgendeinem der Ansprüche von 1 bis 6, ferner umfassend das Festlegen des Zielbereichs (B) im Einzelbild (A).

8. Das Messverfahren nach Anspruch 7, ferner umfassend:
Feststellen, ob der Zielbereich (B) mindestens eine vorgegebene Anzahl von Teilbereichen beinhaltet, die hinsichtlich des Veränderungsbetrags einander ähnlich sind; und
Festlegen des Zielbereichs (B) als Reaktion auf die Feststellung, dass der Zielbereich (B) nicht zumindest die vorgegebene Anzahl von Teilbereichen beinhaltet, die hinsichtlich des Veränderungsbetrags einander ähnlich sind.

9. Das Messverfahren nach Anspruch 7 oder Anspruch 8, ferner umfassend das Schätzen einer Position und einer Größe eines Brustabschnitts oder eines Bauchabschnitts des Subjekts (P) auf der Grundlage einer Position und einer Ausrichtung eines Kopfes des Subjekts (P), wobei
das Festlegen des Zielbereichs (B) das Festlegen eines den Brustabschnitt oder den Bauchabschnitt beinhaltenden Abschnitt als den Zielbereich (B), auf der Grundlage der Position und der Größe des Brustabschnitts oder des Bauchabschnitts beinhaltet.

10. Das Messverfahren nach Anspruch 9, wobei
das Schätzen einer Position und einer Größe das Festlegen der Position und der Größe des Brustabschnitts oder des Bauchabschnitts des Subjekts auf der Grundlage eines Merkmalswerts eines Gesichts des Subjekts (P) beinhaltet.

11. Ein Programm, das von einem auf einem Computer (21) montierten Prozessor (211) ausgeführt wird, wobei das Programm den Computer (21) veranlasst, das Messverfahren nach irgendeinem der Ansprüche von 1 bis 10 durchzuführen.

12. Ein computerlesbares Speichermedium, auf dem ein Programm gespeichert ist, das, wenn es von einem Computer (21) ausgeführt wird, den Computer veranlasst, das Messverfahren nach irgendeinem der Ansprüche von 1 bis 10 durchzuführen.

13. Ein Messsystem (100), das Folgendes umfasst:
eine Kamera (1), die dazu konfiguriert ist, ein Subjekt (P) über einen bestimmten Zeitraum hinweg abzubilden und eine Einzelbildgruppe zu erzeugen; und
eine Verarbeitungsvorrichtung (2), die dazu konfiguriert ist, eine Verarbeitung zur Messung periodischer biologischer Informationen des Subjekts (P) anhand der von der Kamera (1) erzeugten Einzelbildgruppe durchzuführen, wobei
die Verarbeitungsvorrichtung (2) konfiguriert ist, zum:
Unterteilen eines Zielbereichs (B) in jedem der in der Einzelbildgruppe enthaltenen Einzelbilder (A) in eine Vielzahl von Teilbereichen (C, D, E);
Extrahieren eines Veränderungsbetrags (F, G, H) in jedem der Vielzahl von Teilbereichen (C, D, E) aus der Einzelbildgruppe;
Bestimmen von Teilbereichen, aus der Vielzahl von Teilbereichen (C, D, E), die hinsichtlich des Veränderungsbetrags (F, G, H) einander ähnlich sind, wobei Teilbereiche, deren Veränderungsbetrag zwischen den Einzelbildern gleich oder kleiner als ein vorgegebener Wert ist, von der Verarbeitung zur Bestimmung einer Teilbereichsgruppe ausgeschlossen werden; und
Erhalten der periodischen biologischen Information des Subjekts (P) auf der Grundlage eines Summenwerts (I) des Veränderungsbetrags in jedem der ähnlichen Teilbereiche.

## Revendications

1. Un procédé de mesure mis en œuvre par ordinateur, destiné à mesurer des informations biologiques périodiques à partir d'un groupe d'images de trames obtenu par imagerie d'un sujet (P) au cours du temps, le procédé de mesure comprenant :
la division d'une région cible (B) dans chaque image de trame (A) incluse dans le groupe d'images de trames en une pluralité de sous-régions (C, D, E) ;
l'extraction d'une quantité de variation (F, G, H) dans chacune de la pluralité de sous-régions (C, D, E) à partir du groupe d'images de trames ;
la détermination, parmi la pluralité de sous-régions (C, D, E), de sous-régions similaires les unes aux autres en termes de quantité de variation (F, G, H), sachant que des sous-régions dont la quantité de variation entre les images de trames est égale ou inférieure à une valeur prescrite, sont exclues du traitement destiné à déterminer un groupe de sous-régions ; et
l'obtention des informations biologiques périodiques du sujet (P) sur la base d'une valeur de somme de la quantité de variation (I) dans chacune des sous-régions similaires.

2. Le procédé de mesure d'après la revendication 1, sachant que
l'image de trame (A) inclut le sujet (P) qui dort.

3. Le procédé de mesure d'après la revendication 1, sachant que
l'image de trame (A) inclut le sujet au repos.

4. Le procédé de mesure d'après l'une quelconque des revendications de 1 à 3, sachant que
les informations biologiques sont des informations respiratoires.

5. Le procédé de mesure d'après la revendication 4, sachant que
les informations respiratoires comprennent au moins l'un des éléments suivants : une fréquence respiratoire, une durée de respiration, une durée d'expiration, une durée d'inspiration, une amplitude, une période allant de l'expiration au début de l'inspiration et un volume courant.

6. Le procédé de mesure d'après l'une quelconque des revendications de 1 à 5, sachant que
la sous-région (C, D, E) est composée d'un pixel ou d'une pluralité de pixels.

7. Le procédé de mesure d'après l'une quelconque des revendications de 1 à 6, comprenant en outre la définition de la région cible (B) dans l'image de trame (A).

8. Le procédé de mesure d'après la revendication 7, comprenant en outre :
la détermination visant à déterminer si la région cible (B) inclut au moins un nombre prescrit de sous-régions similaires en termes de quantité de variation ; et
la définition de la région cible (B) en réponse à la détermination que la région cible (B) ne comprend pas au moins le nombre prescrit de sous-régions similaires en termes de quantité de variation.

9. Le procédé de mesure d'après la revendication 7 ou la revendication 8, comprenant en outre l'estimation d'une position et d'une taille d'une partie thoracique ou d'une partie abdominale du sujet (P) sur la base d'une position et d'une orientation de la tête du sujet (P), sachant que
la définition de la région cible (B) inclut la définition, en tant que région cible (B), d'une région incluant la partie thoracique ou la partie abdominale sur la base de la position et de la taille de la partie thoracique ou de la partie abdominale.

10. Le procédé de mesure d'après la revendication 9, sachant que
l'estimation d'une position et d'une taille inclut la définition de la position et de la taille de la partie thoracique ou de la partie abdominale du sujet sur la base d'une valeur de caractéristique d'un visage du sujet (P).

11. Un programme exécuté par un processeur (211) monté sur un ordinateur (21), le programme amenant l'ordinateur (21) à effectuer le procédé de mesure d'après l'une quelconque des revendications de 1 à 10.

12. Un support de stockage lisible par ordinateur stockant un programme qui, lorsqu'il est exécuté par un ordinateur (21), amène l'ordinateur à effectuer le procédé de mesure d'après l'une quelconque des revendications de 1 à 10.

13. Un système de mesure (100) comprenant :
une caméra (1) configurée pour imager un sujet (P) au cours du temps et pour générer un groupe d'images de trames ; et
un dispositif de traitement (2) configuré pour effectuer un traitement destiné à mesurer des informations biologiques périodiques du sujet (P) à partir du groupe d'images de trames généré par la caméra (1), sachant que
le dispositif de traitement (2) est configuré pour :
diviser une région cible (B) dans chaque image de trame (A) incluse dans le groupe d'images de trames en une pluralité de sous-régions (C, D, E) ;
extraire une quantité de variation (F, G, H) dans chacune de la pluralité de sous-régions (C, D, E) à partir du groupe d'images de trames ;
déterminer, parmi la pluralité de sous-régions (C, D, E), des sous-régions similaires les unes aux autres en termes de quantité de variation (F, G, H), sachant que des sous-régions dont la quantité de variation entre les images de trames est égale ou inférieure à une valeur prescrite, sont exclues du traitement destiné à déterminer un groupe de sous-régions ; et
obtenir les informations biologiques périodiques du sujet (P) sur la base d'une valeur de somme (I) de la quantité de variation dans chacune des sous-régions similaires.
